# EUROPÄISCHE PATENTSCHRIFT

(11) **EP 3 575 463 B1**
(45) Veröffentlichungstag und Bekanntmachung des Hinweises auf die Patenterteilung: **05.04.2023**
(21) Anmeldenummer: 18020244.2
(22) Anmeldetag: 01.06.2018
(51) Int. Cl.: D04B 1/18, A41B 11/00, A41B 11/02, A61F 13/06, A61F 13/08, D04B 1/26

(54) **BEINBEKLEIDUNGSSTÜCK, INSBESONDERE STRUMPF**
CLOTHING ITEM FOR LEGS, IN PARTICULAR SOCKS
PIÈCE DE VÊTEMENT POUR JAMBES, EN PARTICULIER BAS

(43) Veröffentlichungstag der Anmeldung: 04.12.2019
(73) Patentinhaber: medi GmbH & Co. KG, 95448 Bayreuth (DE)
(72) Erfinder: Hörath, Bernd, 95482 Gefrees (DE)

(56) Entgegenhaltungen:
- CN-A- 106 726 149
- DE-A1-102012 216 180
- US-A1- 2010 130 903
- US-B1- 7 434 423

## Beschreibung

Die vorliegende Erfindung bezieht sich auf ein Beinbekleidungsstück, insbesondere Strumpf, bestehend aus zumindest einem kompressiven Grundgestrickteil nach dem Oberbegriff des Anspruchs 1.

Derartige Beinbekleidungsstücke für den medizinischen Einsatz und/ oder für die Anwendung bei sportlichen Aktivitäten dienen dazu gezielt Druck auf den Körper eines Patienten auszuüben. Der auf den Körper eines Patienten ausgeübte Druck wird als Kompression bezeichnet. Ziel bei Kompressionsstrümpfen beispielsweise, insbesondere in Form von Beinlingen mit oder ohne Hand- oder Fußteil für den medizinischen Einsatz, ist es unter anderem ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von Kompressionsstrümpfen in dem Sportbereich bewirken diese eine Leistungssteigerung bzw. eine verbesserte Regeneration. Insbesondere durch die Wadenmuskulatur (Musculus gastrocnemius), welche bei jeder Aktivität von innen gegen den kompressiven Strumpf mit hoher Stützleistung drückt, werden diese Effekte erzielt. Die Venen, die zwischen der Wadenmuskulatur und dem Strumpf liegen, werden bei jeder Muskelbewegung ausgepresst, was eine verbesserte Blutzirkulation zur Folge hat.

Zur Ausbildung dieser kompressiven Beinbekleidungsstücke, werden diese mittels einer Rundstrickmaschine oder mittels einer ein vorderes und ein hinteres Nadelbett aufweisenden Flachstrickmaschine flach- oder rundgestrickt. Hierzu werden die Gestricke aus wenigstens einem maschenbildenden Strickfaden und einem eingelegten elastischen Schussfaden gestrickt. Zur Herstellung, insbesondere zur Messung und Gütesicherung, von kompressiven Arm- oder Beinstrümpfen für die medizinische Anwendung, existiert die Norm RAL-GZ 387 der Gütezeichengemeinschaft. Aus den Prüfbestimmungen der RAL kann entnommen werden, wie der Druck eines Kompressionsstrumpfes auf ein Bein zu bestimmen ist. Als Messmittel, insbesondere Kompressionsprüfgerät, wird die Prüfung am HOSY Messgerät (Institut Hohenstein) vorgeschlagen. Die Prüfung erfolgt anhand der Messung der Spannkraft in den mehreren Messpunkten, welche abhängig von der jeweiligen Dehnbarkeit des Gestricks, also abhängig von der Elastizität des Gestricks, variiert. Aus der Spannkraft wird die Kompression errechnet. D.h. je mehr Elastizität ein Gestrick in Gestrickquerrichtung aufweist, desto geringer ist die durch das Gestrick am Patienten ausgeübte Kompression. Andererseits je geringer die Elastizität des Gestrickteils ist, desto höher ist die am Patienten ausgeübte Kompression.

Aus dem Stand der Technik sind eine Vielzahl von kompressiven Beinbekleidungsstücken, insbesondere in Form von Strümpfen, bekannt. Ein kompressiver Strumpf, welcher die Aktivität der Wadenmuskulatur berücksichtigt, ist beispielsweise aus der EP 1 240 880 B1 bekannt.

Diese bekannte therapeutische elastische Umhüllung in Form eines Strumpfs aus elastischem Gewebe ist derart ausgebildet, dass bei Gebrauch wenigstens der Unterschenkel eines Benutzers umgeben ist, wobei der Strumpf einen weniger elastischen Abschnitt umfasst, der derart auf der Rückseite des Strumpfs positioniert ist, dass er sich bei Benutzung im Wesentlichen von 1/3 bis zu 2/3 der Höhe des Unterschenkels des Benutzers erstreckt, wo sich während der Benutzung des Strumpfs am Bein sich der Wadenmuskel unter der Haut des Benutzers befinden. Durch die in den Strumpf eingearbeitete unelastische Zone werden durch die Aktivität des Wadenmuskels hohe Druckimpulse erzeugt, welche die Blutzirkulation verstärken.

Auch die US 7,434,423 B1 offenbart einen derartigen Aufbau eines kompressiven Strumpfes, bei dem im Wadenbereich ein weniger elastischer Abschnitt eingearbeitet ist, um lokal die kompressiven Eigenschaften des Strumpfes zu erhöhen. Hierzu werden in den Abschnitt Fanghenkel eingestrickt, welche die Elastizität eines Gestricks verringern und dadurch die kompressive Eigenschaft erhöhen.

Ebenso ist aus der CN 106 726 149 A ein derartiges kompressives Beinbekleidungsstück bekannt. Insbesondere zur Erhöhung der Leistung der Wadenmuskulaturpumpe und zur Optimierung der Druckverteilung sind unterschiedlich elastische und unelastische Bereiche vorgesehen. Zwei voneinander beabstandete, streifenförmige und unelastische Bereiche erstrecken sich hierbei über die Muskelköpfe der Wadenmuskulaturpumpe entlang der Rückseite des Beinbekleidungsstücks.

Darüber hinaus ist es bekannt, derartige kompressive Bekleidungsstücke mit Polsterungen zu versehen. Diese dienen insbesondere dazu, das damit bedeckte Körperteil thermisch zu isolieren und/ oder dieses gegenüber Krafteinwirkung zu schützen. Auch eine Abfederung des Körperteils, beispielsweise bei Anordnung der Polsterung im Bereich der Fußsohle, kann damit erfolgen.

Die DE 10 2012 216180 A1 offenbart beispielsweise ein derartiges kompressives Beinbekleidungsstück, bei welchem ein Fußbereich mit einer Fußpolsterung versehen ist, welche sich vom Zehenbereich bis zu dem Fersenbereich sowie über einen Überzehenbereich erstreckt. Ferner kann eine Polsterung für das untere Ende der Achillessehne vorgesehen sein, die längs eines oberen Randes der Fußpolsterung angrenzt und sich von dort über das untere Ende der Achillessehne erstreckt.

Die US 2010/0130903 A1 offenbart ferner ein kompressives Kleidungsstück in Form einer Hose, wobei zur thermischen Isolierung das Kleidungsstück eine Polsterung in Form von an der Innenseite gleichmäßig angeordneten und quadratförmig ausgebildeten Polsterelementen aufweist. Die Polsterung mit den mehreren Polstererhebungen erstreckt sich in Umfangsrichtung über den gesamten Umfang des Beinbekleidu ngsstücks.

Als nachteilig der aus dem Stand der Technik bekannten Ausgestaltung der Beinbekleidungsstücke erweist sich insbesondere die oftmals schlechte Passform der rundgestrickten kompressiven Gestrickteile sowie der damit verbundene mangelhafte Tragekomfort. Insbesondere bei Sportlern mit einer ausgeprägten Wadenmuskulatur treten der mediale und laterale Muskelkopf des Wadenmuskels, welche beidseits am unteren Teil des Oberschenkelknochens entspringen, stark hervor, so dass der Querschnitt des Unterschenkels stark von einem kreisförmigen Querschnitt abweicht. Die hervortretenden Muskelköpfe drücken somit stark in das Gestrickinnere der rundgestrickten kompressiven Strümpfe. Dies hat zur Folge, dass das kompressive Gestrick, insbesondere die an der Innenseite des Gestricks offen liegenden Schussfäden Reizungen an der Haut, insbesondere im Bereich der Köpfe des Wadenmuskels, aber auch im Bereich des Schienbeins verursacht.

Ein weiterer Nachteil der aus dem Stand der Technik bekannten kompressiven Beinbekleidungsstücke ist die, durch die starke Abweichung des Beins von einem kreisförmigen Querschnitt, mangelhafte Druckverteilung der kompressiven Gestricke auf das Bein. Eine gleichmäßige Druckverteilung wird durch die bekannten Strümpfe nicht gewährleistet. Diese würde nur bei einem Bein mit einem kreisförmigen Querschnitt erzielt werden. Der Querschnitt weicht jedoch in der Praxis, insbesondere bei Sportlern, stark von einem Kreis ab, so dass eine möglichst gleichmäßige Druckverteilung nicht erreicht werden kann. Dies hat zur Folge, dass ein sehr ungleichmäßiges, für den Träger unangenehmes, Druckprofil in Umfangsrichtung entsteht.

Auch werden, durch die von einem kreisförmigen Querschnitt abweichende Geometrie eines Beines, Muster, Bilder, Schriftzüge oder ähnliches, welche auf den Strümpfen oftmals zu Werbezwecken aufgebracht und/ oder eingearbeitet sind, verzerrt dargestellt. Dies stellt ebenfalls einen Nachteil aus dem Stand der Technik dar.

Der vorliegenden Erfindung liegt daher die Aufgabe zugrunde ein Beinbekleidungsstück, insbesondere Strumpf mit einem kompressiven Gestrickteil, zu schaffen, welches die Nachteile aus dem Stand der Technik vermeidet, insbesondere die Passform und den Tragekomfort des Beinbekleidungsstücks, die Druckverteilung auf das Bein sowie die Optik des Kleidungsstücks, insbesondere im getragenen Zustand, verbessert.

Entsprechend der erfindungsgemäßen Ausführung des Beinbekleidungsstück, insbesondere als Strumpf, Beinling, Legging oder Hose, besteht dieser bzw. diese aus zumindest einem kompressiven Grundgestrickteil, welches an der Innenseite zumindest eine erste Polsterung aufweist, wobei die zumindest eine erste Polsterung derart ausgebildet ist, dass diese im getragenen Zustand des Beinbekleidungsstücks zwischen den beiden Muskelköpfen des Musculus gastrocnemius angeordnet ist. Die Polsterung erstreckt sich hierbei also zwischen den beiden Muskelköpfen des Musculus gastrocnemius ohne diese zu überlappen. Die Innenseite des Beinbekleidungsstücks wird somit partiell in Umfangsrichtung gesehen, nach innen versetzt. Durch die Polsterung wird somit eine von einem kreisförmigen Querschnitt abweichende Geometrie des Beines ausgeglichen, sowie unter anderem auch ein durch die Muskelköpfe gegebenenfalls gebildeter Hohlraum zwischen Beinaußenseite und Beinbekleidungsstück gefüllt. Die Polsterung besteht bevorzugt aus einem weichen für den Anwender angenehmen und kaum spürbaren Material, ohne jegliche reizproduzierende Elemente. Zudem erstreckt sich die zumindest eine erste Polsterung im getragenen Zustand des Beinbekleidungsstücks zusätzlich entlang der Achillessehne und überlappt diese. Die Breite der zumindest einen ersten Polsterung in Gestrickumfangsrichtung beträgt hierbei vorzugsweise zwischen 1 cm und 2 cm, maximal 4 cm.

Gemäß einem Ausführungsbeispiel des Beinbekleidungsstück, insbesondere Strumpf, weist dieses zumindest eine zweite Polsterung auf, die im getragenen Zustand des Beinbekleidungsstücks sich zumindest teilweise entlang dem Schienbeinknochen erstreckt und diesen überlappt. Alternativ oder zusätzlich ist es auch möglich, dass die Polsterung im getragenen Zustand des Beinbekleidungsstücks sich auch links und rechts neben dem Schienbein an dem Grundgestrickteil angeordnet ist. Beide Varianten führen dazu, dass ein möglichst kreisförmiger Querschnitt für das Grundgestrickteil erzielt wird.

Gemäß einem weiteren Ausführungsbeispiel des Beinbekleidungsstück, insbesondere Strumpf, weist dieses ein Fußteil, bestehend aus einem zweiten, vorzugsweise kompressiven Grundgestrickteil, auf, wobei dieses zumindest eine dritte Polsterung für den Zehenbereich, die Ferse und/ oder die Fußsohle umfasst.

Die erste, zweite und/ oder dritte Polsterung sind bevorzugt durch einen gestrickten Abschnitt in den Grundgestrickteilen ausgebildet, der sich bezüglich seiner Bindungsart von einem an die Polsterung angrenzenden Bereich der Grundgestrickteile unterscheidet. Hierbei sind die mehreren Polsterungen bevorzugt als ein Plüsch, insbesondere Sandwich-Plüsch, ausgebildet. Alternativ ist es möglich, dass die erste, zweite und/ oder dritte Polsterung durch ein von den Grundgestrickteilen getrenntes Polsterelement ausgebildet ist, das lösbar oder unlösbar mit den Grundgestrickteilen verbindbar ist.

Die Breite der zweiten Polsterung beträgt in Gestrickumfangsrichtung, vorzugsweise ebenfalls zwischen 1 cm und 2 cm, maximal 4 cm. Besonderes bevorzugt ist die erste Polsterung derart ausgebildet, dass diese der Kontur der Außenseite der hervortretenden Muskelköpfe, insbesondere im Bereich zwischen den beiden Köpfen folgt. Im einfachsten Fall weist die Polsterung einen stegförmigen Querschnitt auf.

Das Grundgestrickteil ist bevorzugt durch einen oder mehrere maschenbildende Grundstrickfäden sowie zumindest einen eingelegten Schussfaden gebildet, um eine kompressives Grundgestrickteil auszubilden. Bevorzugt weist das Grundgestrickteil sowie die erste und/ oder zweite Polsterung in Gestricklängsrichtung des Beinbekleidungsstücks einen graduellen Kompressionsverlauf auf. Die Kompressionsstärke nimmt hierbei bevorzugt beginnend von der Fessel in Richtung der Wadenmuskulatur ab oder zu. Besonderes bevorzugt betragen die durch das erste und zweite Grundgestrickteil erzeugten kompressiven Drücke in einem Fesselbereich des Beinbekleidungsstücks zwischen 10 und 30 mmHg, im Wadenbereich zwischen 5 und 20 mmHg und in einem Mittelfußbereich zwischen 10 und 30 mmHg.

Gemäß einem weiteren Ausführungsbeispiel des Beinbekleidungsstück, insbesondere Strumpf, weist das erste und/ oder zweite Grundgestrickteil des Beinbekleidungsstücks eine oder mehrere Zonen mit unterschiedlichen Elastizitäten in Längs- und/ oder Umfangsrichtung auf. Insbesondere überlappt hierbei eine erste Zone mit erhöhter Elastizität in Längs- und/ oder Umfangsrichtung im getragenen Zustand des Beinbekleidungsstück den Malleolus, um eine Entlastungszone für diesen auszubilden. Zusätzlich oder alternativ überlappt eine zweite Zone mit erhöhter oder reduzierter Elastizität, im Vergleich zum restlichen bzw. angrenzenden Grundgestrick, in Längs- und/ oder Umfangsrichtung im getragenen Zustand des Beinbekleidungsstück die beiden Muskelköpfe des Musculus gastrocnemius.

Gemäß einem weiteren Ausführungsbeispiel des Beinbekleidungsstück, insbesondere Strumpf, weist dieses neben dem ersten Grundgestrickteil zumindest einen zweilagigen Bund auf, wobei die zumindest eine zweite sich entlang dem Schienbeinknochen erstreckende Polsterung sich zumindest teilweise in den Bund, vorzugsweise zwischen die erste und zweite Lage des Bundes, erstreckt. Hierdurch wird eine zusätzliche Polsterung im Bereich des Schienbeinknochens erzielt, was bei einem Bund mit besonders viel Kompression, um einen sicheren Halt des Strumpfes, insbesondere Wadenstrumpfes, am Bein zu gewährleisten, besonders von Vorteil ist.

Das vorliegende Beinbekleidungsstück zeichnet sich durch eine Reihe erheblicher Vorteile aus.

Durch die Ausbildung des Beinbekleidungsstück mit einer Polsterung zwischen den beiden Muskelköpfen des Musculus gastrocnemius wird der Tragekomfort des Beinbekleidungsstückes, insbesondere des kompressiven Strumpfes, wesentlich erhöht. Ein unerwünschter lokaler Druckanstieg in Umfangsrichtung, wie er aus dem Stand der Technik bekannt ist, auf Grund der teilweise starken geometrischen Abweichung des Beines eines Trägers von einem kreisförmigen Querschnitt, und welche beim Tragen der Kleidungsstücke für den Patienten als störend empfunden wird, wird durch das erfindungsgemäße Beinbekleidungsstück vermieden.

Es wird ferner durch die Auspolsterung von sogenannten Vertiefungen bzw. Rücksprüngen in der Oberfläche des Beines eines Trägers des Beinbekleidungsstücks eine möglichst gleichmäßige Kompressionswirkung erzeugt. Was ebenfalls einen wesentlichen Vorteil bildet.

Einen weiteren Vorteil der Erfindung bildet die Möglichkeit Muster, Bilder, Schriftzüge oder ähnliches, welche auf den Strümpfen oftmals zu Werbezwecken aufgebracht und/ oder eingearbeitet sind, korrekt, also ohne jegliche Verzerrungen, bedingt durch die von einem kreisförmigen Querschnitt abweichende Geometrie des Beines eines Trägers, darzustellen. Durch die lokal begrenzten Polsterungen, insbesondere zwischen den hervortretenden Stellen, wie Schienbeinknochen oder Köpfe der Wadenmuskulatur, wird ein möglichst kreisförmiger Querschnitt erzielt.

Nachfolgend wird die Erfindung anhand mehrerer Ausführungsbeispiele und in Verbindung mit den beigefügten Zeichnungen erläutert.

Dabei zeigt:
Figur 1 die Anatomie des Unterschenkels, insbesondere den Wadenmuskel Musculus gastrocnemius sowie dessen medialen und lateralen Muskelkopf,
Figur 2 ein Ausführungsbeispiel des Beinbekleidungsstücks, insbesondere eines kompressiven Wadenstrumpfes, von der Rückseite,
Figur 3 das Beinbekleidungsstück, insbesondere den kompressiven Wadenstrumpf, aus

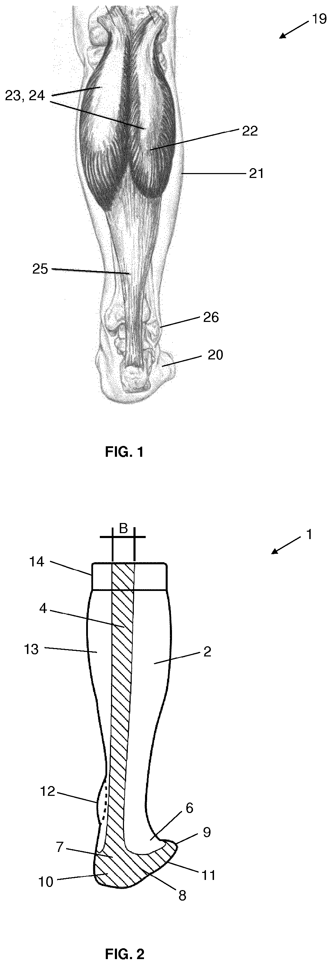

Figur 2 in einer Vorderansicht,

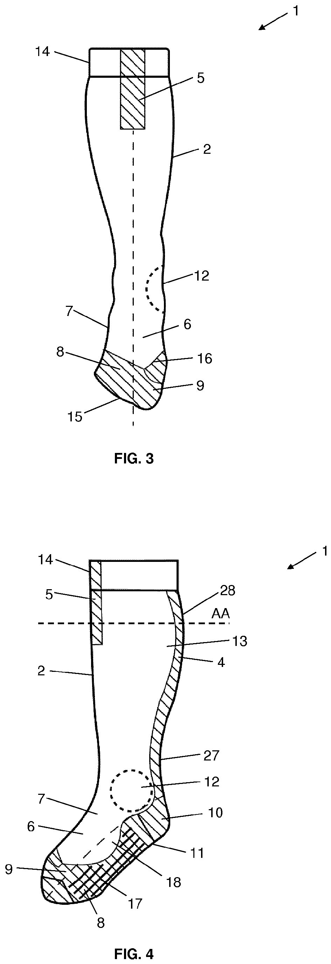

Figur 4 den kompressiven Wadenstrumpf, aus den Figuren 2 und 3 in einer Seitenansicht, sowie

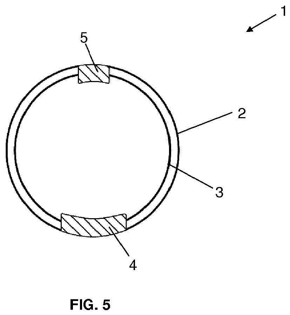

Figur 5 einen Querschnitt durch den kompressiven Wadenstrumpf entlang der Linie AA aus Figur 4;

Figur 1 zeigt zur Verdeutlichung der Erfindung die Anatomie des menschlichen Beines 19, insbesondere den Unterschenkel 21. Dieser umfasst insbesondere den Wadenmuskel Musculus gastrocnemius 22, welcher wiederum die Muskelköpfe, nämlich den medialen und lateralen Muskelkopf 23, 24, aufweist. Vom unteren Ende des Wadenmuskels 22 erstreckt sich die Achillessehne 25 bis in einen Fußbereich 20. Ebenso deutlich gezeigt ist der Fußknöchel Malleolus 26, welcher, wie die beiden Köpfe 23, 24 des Wadenmuskels 22, hervortritt. Insbesondere bei Sportlern mit einer ausgeprägten Wadenmuskulatur 22 treten der mediale und laterale Muskelkopf 23, 24 sehr stark hervor, so dass dadurch der Querschnitt des Unterschenkels stark von einem kreisförmigen Querschnitt abweicht und ein angelegter Wadenstrumpf lokal sehr stark verdehnt wird.

In Figur 2 ist ein Ausführungsbeispiel des erfindungsgemäßen Beinbekleidungsstücks 1, insbesondere eines kompressiven Wadenstrumpfes, von der Rückseite gezeigt. Dieser besteht aus zumindest einem ersten bevorzugt mittels eine Rundstrickmaschine gestrickten kompressiven Grundgestrickteil 2. An der Innenseite weist dieses zumindest eine erste Polsterung 4 auf, welche derart ausgebildet ist, dass diese im getragenen Zustand des Beinbekleidungsstücks 1 zwischen den beiden Muskelköpfen 23, 24 des Musculus gastrocnemius 22 angeordnet ist. Die Polsterung 4 erstreckt sich hierbei in Umfangsrichtung gesehen nur zwischen den beiden Muskelköpfen 23, 24 ohne diese zu überlappen. Die Polsterung 4, gefertigt aus einem weichen für den Anwender kaum spürbaren Material, dient dazu eine von einem kreisförmigen Querschnitt abweichende Geometrie des Beines 19 auszugleichen. Die Polsterung 4 kann, wie gezeigt, streifenförmig sein, aber auch andere Geometrien aufweisen. Beispielsweise wäre eine Ausbildung als Viereck mit zwei nach innen gekrümmten seitlichen Außenkanten, zur Anpassung an die Muskelköpfe 23, 24, möglich. Weitere Formen sind denkbar. Die Breite B der ersten Polsterung 4 beträgt in Gestrickumfangsrichtung gesehen vorzugsweise zwischen 1 cm und 2 cm, maximal jedoch 4 cm. Bevorzugt erstreckt sich die zumindest eine erste Polsterung 4, wie gezeigt, im getragenen Zustand des Beinbekleidungsstücks 1 zusätzlich zumindest teilweise entlang der Achillessehne 25 in Richtung Ferse und überlappt die Sehne 25.

Des Weiteren weist der Strumpf 1 ein Fußteil 6 auf, welches aus einem zweiten, vorzugsweise kompressiven Grundgestrickteil 7, besteht. Dieses Fußteil 6 umfasst eine weitere Polsterung 8 für den Zehenbereich 9, die Ferse 10 und die Fußsohle 11. Die Polsterung 8 erstreckt sich in diesem Ausführungsbeispiel von der Sohle 11 bis in die Seiten des Fußteils 6 hinauf.

In diesem Ausführungsbeispiel sind die mehreren Polsterungen 4 und 8 durch einen gestrickten Abschnitt in den Grundgestrickteilen 2, 7 ausgebildet, der sich bezüglich seiner Bindungsart von einem an die Polsterung 4, 8 angrenzenden Bereich der Grundgestrickteile 2, 7 unterscheidet. Hierbei sind die mehreren Polsterungen 4, 8 bevorzugt als ein Plüsch, insbesondere Sandwich-Plüsch, ausgebildet. Hierbei weist das Grundgestrick der Grundgestrickteile 2, 7 einen oder mehrere maschenbildende Grundstrickfäden auf, in denen zumindest ein kompressionsgebender Schussfaden eingelegt ist und zumindest ein Plüschfaden pro Maschenreihe auf den Grundstrickfaden aufplattiert ist. D.h. der Plüschfaden ist zusätzlich zu dem Grundstrickfaden und dem Schussfaden in das Grundgestrickteil 2, 7 eingearbeitet und bildet durch seine Verarbeitung eine weitere hervortretende Gestrickebene, welche je nach Einstellung der Strickmaschine unterschiedlich hoch ausgebildet werden kann. Ferner erstreckt sich der Plüschfaden bevorzugt durch das Grundgestrick bis an die Außenseite des Grundgestrickteils 2, so dass durch verschiedenfarbige Strick- oder Plüschfäden verschiedenste Farbgestaltungen an der Außenseite des Beinbekleidungsstücks 1 möglich sind. Durch die Auswahl eines weichen voluminösen Plüschfadens kann eine besonders weiche Polsterung 4, 8 ausgebildet werden. Das Grundgestrickteil 2 sowie die erste Polsterung 4 weist in dem gezeigten Ausführungsbeispiel in Gestricklängsrichtung des Beinbekleidungsstücks 1 einen graduellen Kompressionsverlauf auf. Die Kompressionsstärke nimmt hierbei vom unteren Ende in Richtung der Wadenmuskulatur 22 ab. Durch die Verwendung einer Plüschung als Polsterung wird der Kompressionsverlauf des Beinbekleidungsstücks 1 nicht negativ beeinflusst.

Des Weiteren weist der gezeigte Strumpf 1 zwei Zonen 12, 13 mit unterschiedlichen Elastizitäten in Längs- und/ oder Umfangsrichtung auf. Die erste Zone 12 mit erhöhter Elastizität in Längs- und/ oder Umfangsrichtung überlappt im getragenen Zustand des Beinbekleidungsstück den Malleolus 26, um eine Entlastungszone für den Malleolus 26 auszubilden. Eine zweite Zone 13 mit reduzierter Elastizität in Längs- und/ oder Umfangsrichtung, im Vergleich zum restlichen bzw. angrenzenden Grundgestrick, überlappt die beiden Muskelköpfe 23, 24 des Muscukus gastrocnemius 22.

Am oberen Ende des ersten Grundgestrickteils 2 weist der Strumpf 1 einen zweilagigen Bund 14 auf, in den sich die rückseitige Polsterung 4 hineinerstreckt. Die Polsterung 4 kann alternativ auch bereits vorzeitig im Grundgestrickteil 14 enden.

In Figur 3 ist das Beinbekleidungsstück 1, insbesondere der kompressive Wadenstrumpf, aus Figur 2 in einer Vorderansicht gezeigt. Dieser umfasst, wie zuvor gezeigt, ein erstes Grundgestrickteil 2, welches den Unterschenkel 21 umgibt, sowie ein zweites als Fußteil 6 ausgebildetes Grundgestrickteil 7. Hierbei ist eine Polsterung 5 gezeigt, die im getragenen Zustand des Beinbekleidungsstücks 1 sich zumindest teilweise entlang dem Schienbeinknochen erstreckt und diesen überlappt. Die Polsterung 5 ist ebenfalls bevorzugt, wie zuvor beschrieben, mittels einer Plüschung ausgebildet, welche in das Grundgestrickteil 2 eingearbeitet ist. Zusätzlich erstreckt sich die Polsterung 5 in den Bund 14. Der Bund 14 ist bevorzugt zweilagig ausgebildet, wobei die sich entlang dem Schienbeinknochen erstreckende Polsterung 5 sich bevorzugt zumindest teilweise zwischen die erste und zweite Lage des Bundes 14 erstreckt, um so eine möglichst nicht sichtbare Polsterung auch in dem Bundbereich für den Schienbeinknochen bereitzustellen.

Das Fußteil 6 weist an seinem vorderen Ende eine flache Übergangsnaht 16 zwischen Mittelfußbereich 18 und Zehenbereich 9 auf. Der Zehenbereich 9 sowie zumindest Teile des Mittelfußbereichs sind bevorzugt mittels der bereits in Figur 2 beschriebenen Polsterung 8 an der Innenseite des Beinbekleidungsstücks 1 gepolstert. Der Strumpf 1 selbst ist bevorzugt mit einer asymmetrischen Fußspitze 15 gestrickt. D.h. das Ende des Strumpfes 1 ist an den Verlauf der Zehen angepasst. Ebenfalls gezeigt, dieses mal von vorne, ist die Zone 12 mit erhöhter Elastizität in Längs- und/ oder Umfangsrichtung zur Aufnahme des Malleolus.

In Figur 4 ist das Beinbekleidungsstück 1 aus den Figuren 2 und 3 schließlich in der Seitenansicht gezeigt. Wie zuvor beschrieben ist der Strumpf 1 aus einem Stück bevorzugt rundgestrickt und weist mehrere Abschnitte, nämlich ein erstes und zweites Grundgestrickteil 2, 7 sowie einen Bund 14 auf. Ebenso gezeigt sind die mehreren Polsterungen 4, 5 und 8. Die rückseitige Polsterung 4 erstreckt zwischen den beiden Köpfen 23, 24 des Wadenmuskels 21 entlang der Achillessehne 25 bis in die Polsterung 8 des Fußteils 6, welche einen Zehenbereich 9, die Fußsohle 11, sowie die Ferse 10 umgibt. Der Übergangsbereich zwischen Polsterung 8 und 4 ist derart ausgebildet, dass eine Zone 12 für den Malleolus ausgespart bzw. ausgenommen ist. Hier soll keine weitere auftragende Schicht an der Innenseite des Strumpfes 1 vorhanden sein. Im Sohlenreich 11 sind ferner bevorzugt ein oder mehrere bezüglich der Gestrickaußenseite zurückversetzte Längsrippen 17 vorgesehen. Diese werden durch eine vom restlichen Grundgestrick unterschiedliche Bindungsart erzeugt und dienen zur besseren Griffigkeit des Strumpfes 1 an dessen Unterseite. Neben der Polsterung 4 weist der Strumpf 1 im Wadenbereich bevorzugt eine Zone 13 mit reduzierter Elastizität in Längs- und/ oder Umfangsrichtung auf, die im getragenen Zustand des Beinbekleidungsstück 1 die beiden Muskelköpfe 23, 24 des Musculus gastrocnemius 22 überlappt. Hierdurch werden, neben der durch die mehreren Polsterungen 4, 5 erzielten gleichmäßigeren Druckverteilung, gleichzeitig höhere Druckimpulse durch die Aktivität des Wadenmuskels erzeugt, welche die Blutzirkulation verstärken.

Die durch das Beinbekleidungsstück 1 erzeugten kompressiven Drücke liegen bevorzugt in einem Fesselbereich 27 zwischen 10 und 30 mmHg, im Wadenbereich 28 zwischen 5 und 20 mmHg und in einem Mittelfußbereich 18 zwischen 10 und 30 mmHg. Die Werte werden mittels der eingangs vorgestellten Messmethodik und Messgerät, insbesondere mittels der Prüfung am HOSY Messgerät (Institut Hohenstein), gemessen.

In Figur 5 ist schließlich ein Querschnitt durch das Beinbekleidungsstück 1, insbesondere durch den kompressiven Wadenstrumpf 1, entlang der Linie AA aus Figur 4 gezeigt. Veranschaulicht wird die sich an der Innenseite 3 des ersten Grundgestrickteil 2 erstreckende ersten Polsterung 4 zur Positionierung zwischen den beiden Köpfen 23, 24 des Wadenmuskels 22 sowie zweite Polsterung 5 zur Aufnahme des Schienbeinknochens. Die Polsterungen 4, 5 können, wie zuvor beschrieben, in Umfangsrichtung unterschiedlichste geometrische Formen aufweisen. Im einfachsten Fall sind die Polsterungen 4, 5 stegförmig ausgebildet. Idealerweise weist die rückseitige Polsterung 4 in Umfangsrichtung eine Kontur auf, wie ebenfalls zuvor beschrieben, die dem Verlauf der Muskelköpfe 23, 24 entspricht.

Parallel oder alternativ dazu können die Polsterungen 4, 5 jeweils natürlich auch unterschiedliche Höhen aufweisen. So ist bevorzugt bei der Polsterung 5 der randseitige Bereich höher als der mittlere Bereich ausgebildet, um den hervorstehenden Schienbeinknochen des Beins eines Trägers aufzunehmen. Die unterschiedlichen Höhen der Polsterungen 4, 5 können durch geeignete Polsterelemente, welche entsprechend zugeschnitten werden, oder auch durch unterschiedliche Plüschungen mit unterschiedlich hohen Plüschhenkeln erzeugt werden. So sind in einem ersten Bereich die Plüschhenkel kürzer oder länger im Vergleich zu einem zweiten angrenzenden Bereich mit Plüsch.

Die Erfindung ist nicht auf die beschriebenen Ausführungsbeispiele beschränkt, sondern umfasst alle Ausführungen, die unter den Schutzumfang der Ansprüche fallen.

## Patentansprüche

1. Beinbekleidungsstück (1), insbesondere Strumpf, bestehend aus zumindest einem kompressiven Grundgestrickteil (2), welches an der Innenseite (3) zumindest eine erste Polsterung (4) aufweist, **dadurch gekennzeichnet, dass** die zumindest eine erste Polsterung (4) derart ausgebildet ist, dass diese im getragenen Zustand des Beinbekleidungsstücks (1) zwischen den beiden Muskelköpfen (23, 24) des Musculus gastrocnemius (22) angeordnet ist, ohne diese zu überlappen, und sich zusätzlich entlang der Achillessehne (25) erstreckt und diese überlappt und dass die Breite (B) der zumindest einen ersten Polsterung (4) in Gestrickumfangsrichtung, vorzugsweise zwischen 1 cm und 2 cm, maximal 4 cm beträgt.

2. Beinbekleidungsstück (1) nach Anspruch 1, **dadurch gekennzeichnet, dass** das Beinbekleidungsstück (1) zumindest eine zweite Polsterung (5) aufweist, die im getragenen Zustand des Beinbekleidungsstücks (1) sich zumindest teilweise entlang dem Schienbeinknochen erstreckt und diesen überlappt.

3. Beinbekleidungsstück (1) nach Anspruch 1 oder 2, **dadurch gekennzeichnet, dass** das Beinbekleidungsstück (1) ein Fußteil (6), bestehend aus einem zweiten, vorzugsweise kompressiven Grundgestrickteil (7), aufweist, wobei dieses zumindest eine dritte Polsterung (8) für den Zehenbereich (9), die Ferse (10) und/ oder die Fußsohle (11) aufweist.

4. Beinbekleidungsstück (1) nach einem der vorherigen Ansprüchen, **dadurch gekennzeichnet, dass** die erste, zweite und/ oder dritte Polsterung (4, 5, 8) durch einen gestrickten Abschnitt in den Grundgestrickteilen (2, 7) ausgebildet ist, der sich bezüglich seiner Bindungsart von einem an die Polsterung (4, 5, 8) angrenzenden Bereich der Grundgestrickteile (2, 7) unterscheidet.

5. Beinbekleidunasstück (1) nach Anspruch 4. **dadurch gekennzeichnet. dass** die erste, zweite und/ oder dritte Polsterung (4, 5, 8) als ein Plüsch insbesondere Sandwich-Plüsch, ausgebildet ist.

6. Beinbekleidungsstück (1) nach einem der vorherigen Ansprüchen, **dadurch gekennzeichnet, dass** die erste, zweite und/ oder dritte Polsterung (4, 5, 8) durch ein von den Grundgestrickteilen (2, 7) getrenntes Polsterelement ausgebildet ist, das lösbar oder unlösbar mit den Grundgestrickteilen (2, 7) verbindbar ist.

7. Beinbekleidungsstück (1) nach Anspruch 2, **dadurch gekennzeichnet, dass** die Breite (B) der zweiten Polsterung (5) in Gestrickumfangsrichtung, vorzugsweise zwischen 1 cm und 2 cm, maximal 4 cm beträgt.

8. Beinbekleidungsstück (1) nach einem der vorherigen Ansprüchen, **dadurch gekennzeichnet, dass** die Grundgestrickteile (2, 7) durch einen oder mehrere maschenbildende Grundstrickfäden sowie zumindest einen eingelegten Schussfaden gebildet ist.

9. Beinbekleidungsstück (1) nach einem der vorherigen Ansprüchen, **dadurch gekennzeichnet, dass** das erste und/ oder zweite Grundgestrickteil (2, 7) sowie die erste und/ oder zweite Polsterung (4, 5) in Gestricklängsrichtung des Beinbekleidungsstücks (1) einen graduellen Kompressionsverlauf aufweist.

10. Beinbekleidungsstück (1) nach Anspruch 9 in seinem Rückbezug auf Anspruch 3, **dadurch gekennzeichnet, dass** die durch das erste und zweite Grundgestrickteil (2, 7) erzeugten kompressiven Drücke in einem Fesselbereich (27) des Beinbekleidungsstücks (1) zwischen 10 und 30 mmHg, im Wadenbereich (28) zwischen 5 und 20 mmHg und in einem Mittelfußbereich (18) zwischen 10 und 30 mmHg betragen.

11. Beinbekleidungsstück (1) nach einem der vorherigen Ansprüche, **dadurch gekennzeichnet, dass** das erste und/ oder zweite Grundgestrickteil (2, 7) des Beinbekleidungsstücks (1) eine oder mehrere Zonen (12, 13) mit unterschiedlichen Elastizitäten in Längs- und/ oder Umfangsrichtung aufweist.

12. Beinbekleidungsstück (1) nach Anspruch 11, **dadurch gekennzeichnet, dass** zumindest eine erste Zone (12) mit erhöhter Elastizität in Längs- und/ oder Umfangsrichtung im getragenen Zustand des Beinbekleidungsstück (1) den Malleolus (26) überlappt.

13. Beinbekleidungsstück (1) nach Anspruch 11 oder 12, **dadurch gekennzeichnet, dass** zumindest eine zweite Zone (13) mit erhöhter oder reduzierter Elastizität in Längs- und/ oder Umfangsrichtung im getragenen Zustand des Beinbekleidungsstück (1) die beiden Muskelköpfe (23, 24) des Musculus gastrocnemius (22) überlappt.

14. Beinbekleidungsstück (1) nach Anspruch 2, **dadurch gekennzeichnet, dass** das Beinbekleidungsstück (1) neben dem ersten Grundgestrickteil (2) zumindest einen zweilagigen Bund (14) aufweist, wobei die zumindest eine zweite sich entlang dem Schienbeinknochen erstreckende Polsterung (5) sich zumindest teilweise in den Bund (14), vorzugsweise zwischen die erste und zweite Lage des Bundes (14) erstreckt.

## Claims

1. A clothing item for legs (1), in particular stocking, consisting of at least one compressive basic knitted part (2) which has at least one first padding (4) on the inside (3), **characterized in that** the at least one first padding (4) is configured in such a way that when the clothing item for legs (1) is worn, this is arranged between the two muscle heads (23, 24) of the gastrocnemius muscle (22) without overlapping this and additionally extends along the Achilles tendon (25) and overlaps this and that the width (B) of the at least one first padding (4) in the circumferential direction of the knitted fabric is preferably between 1 cm and 2 cm, at most 4 cm.

2. The clothing item for legs (1) according to Claim 1, **characterized in that** the clothing item for legs (1) has at least one second padding (5) which, when the clothing item for legs (1) is worn, extends at least partially along the shin bone and overlaps it.

3. The clothing item for legs (1) according to Claim 1 or 2, **characterized in that** the clothing item for legs (1) has a foot part (6), consisting of a second, preferably compressive basic knitted part (7), wherein this has at least a third padding (8) for the toe area (9), the heel (10) and/or the sole (11).

4. The clothing item for legs (1) according to one of the preceding claims, **characterized in that** the first, second and/or third padding (4, 5, 8) is formed by a knitted section in the basic knitted parts (2, 7) which differs in terms of its type of weave from an area of the basic knitted parts (2, 7) adjoining the padding (4, 5, 8).

5. The clothing item for legs (1) according to Claim 4, **characterized in that** the first, second and/or third padding (4, 5, 8) is configured as plush, in particular sandwich plush.

6. The clothing item for legs (1) according to one of the preceding claims, **characterized in that** the first, second and/or third padding (4, 5, 8) is formed by a padding element separate from the basic knitted parts (2, 7), which can be connected detachably or non-detachably to the basic knitted parts (2, 7).

7. The clothing item for legs (1) according to Claim 2, **characterized in that** the width (B) of the second padding (5) in the circumferential direction of the knitted fabric is preferably between 1 cm and 2 cm, at most 4 cm.

8. The clothing item for legs (1) according to one of the preceding claims, **characterized in that** the basic knitted parts (2, 7) are formed by one or more stitch-forming basic knitting threads and at least one inserted weft thread.

9. The clothing item for legs (1) according to one of the preceding claims, **characterized in that** the first and/or second basic knitted part (2, 7) and the first and/or second padding (4, 5) have a gradual compression profile in the longitudinal direction of the knitted fabric of the clothing item for legs (1).

10. The clothing item for legs (1) according to Claim 9 related back to Claim 3, **characterized in that** the compressive pressures generated by the first and second basic knitted part (2, 7) in an ankle area (27) of the clothing item for legs (1) are between 10 and 30 mm Hg, in the calf area (28) between 5 and 20 mm Hg and in a metatarsal region (18) between 10 and 30 mm Hg.

11. The clothing item for legs (1) according to one of the preceding claims, **characterized in that** the first and/or second basic knitted part (2, 7) of the clothing item for legs (1) has one or more zones (12, 13) with different elasticities in the longitudinal and/or circumferential direction.

12. The clothing item for legs (1) according to Claim 11, **characterized in that** at least a first zone (12) with increased elasticity in the longitudinal and/or circumferential direction overlaps the malleolus (26) when the clothing item for legs (1) is worn.

13. The clothing item for legs (1) according to Claim 11 or 12, **characterized in that** at least a second zone (13) with increased or reduced elasticity in the longitudinal and/or circumferential direction when the clothing item for legs (1) is being worn overlaps the two muscle heads (23, 24) of the gastrocnemius muscle (22).

14. The clothing item for legs (1) according to Claim 2, **characterized in that** the clothing item for legs (1) has at least one two-layer band (14) next to the first basic knitted part (2), wherein the at least one second padding (5) extending along the shinbone extends at least partially into the band (14), preferably between the first and second layers of the band (14).

## Revendications

1. Vêtement de jambe (1), notamment chaussette, constitué d'au moins une pièce compressive en tricot de base (2), qui sur la face intérieure (3) comporte au moins un premier bourrelet (4), **caractérisé en ce que** l'au moins un premier bourrelet (4) est conçu de telle sorte que lorsque le vêtement (1) est porté celui-ci se trouve entre les deux chefs musculaires (23, 24) du lambeau musculaire gastrocnémien (22), sans les chevaucher, et s'étende additionnellement le long du tendon d'Achille (25) et chevauche ce dernier, et **en ce que** la largeur (B) de l'au moins un premier bourrelet (4) dans la direction périphérique du tricot soit comprise de préférence entre 1 cm et 2 cm, soit au maximum de 4 cm.

2. Vêtement de jambe (1) selon la revendication 1, **caractérisé en ce que** le vêtement (1) comporte au moins un deuxième bourrelet (5), qui lorsque le vêtement (1) est porté, s'étend au moins partiellement le long du tibia et chevauche ce dernier.

3. Vêtement de jambe (1) selon la revendication 1 ou 2, **caractérisé en ce que** le vêtement (1) comporte un élément de pied (6), constitué d'une deuxième pièce de tricot de base (7), de préférence de compression, cet au moins un troisième bourrelet (8) pour la région des orteils (9) comportant le talon (10) et / ou die plante de pied (11).

4. Vêtement de jambe (1) selon l'une quelconque des revendications précédentes, **caractérisé en ce que** le premier, le deuxième et / le troisième bourrelet (4, 5, 8) est conçu en une partie tricotée dans les pièces de tricot de base (2, 7), qui au niveau de son armure se différencie d'une région de la pièce de tricot de base (2, 7) qui est adjacente au bourrelet (4, 5, 8).

5. Vêtement de jambe (1) selon la revendication 4, **caractérisé en ce que** le premier, le deuxième et / ou le troisième bourrelet (4, 5, 8) est conçu sous la forme d'une peluche, notamment d'une peluche en sandwich.

6. Vêtement de jambe (1) selon l'une quelconque des revendications précédentes, **caractérisé en ce que** le premier, le deuxième et / ou le troisième bourrelet (4, 5, 8) est conçu par un bourrelet séparé des pièces de tricot de base (2, 7), qui est susceptible d'être assemblé de manière amovible ou inamovible avec les pièces de tricot de base (2, 7).

7. Vêtement de jambe (1) selon la revendication 2, **caractérisé en ce que** la largeur (B) du bourrelet (5) dans la direction périphérique du tricot est comprise de préférence entre 1 cm et 2 cm, s'élève au maximum à 4 cm.

8. Vêtement de jambe (1) selon l'une quelconque des revendications précédentes, **caractérisé en ce que** les pièces de base du tricot (2, 7) sont conçues en un ou plusieurs fils de base du tricot formant des mailles, ainsi qu'en au moins un fil de trame inséré.

9. Vêtement de jambe (1) selon l'une quelconque des revendications précédentes, **caractérisé en ce que** la première et / ou la deuxième pièce de tricot de base (2, 7), ainsi que dans la direction longitudinale de tricotage du vêtement (1), le premier et / ou le deuxième bourrelet (4, 5) présente un trajet de compression graduel.

10. Vêtement de jambe (1) selon la revendication 9 dans son renvoi à la revendication 3, **caractérisé en ce que** les pressions de compression générées par la première et la deuxième pièce de tricot de base (2, 7) sont comprises dans une région de cheville (27) du vêtement (1) entre 10 et 30 mmHg, dans la région du mollet (28) entre 5 et 20 mmHg et dans une zone centrale du pied (18) entre 10 et 30 mmHg.

11. Vêtement de jambe (1) selon l'une quelconque des revendications précédentes, **caractérisé en ce que** la première et / ou la deuxième pièce de tricot de base (2, 7) du vêtement (1) comporte une ou plusieurs zones (12, 13) de différentes élasticités dans la direction longitudinale et / ou périphérique.

12. Vêtement de jambe (1) selon la revendication 11, **caractérisé en ce que** lorsque le vêtement (1) est porté, au moins une première zone (12) d'élasticité accrue chevauche la malléole (26) dans la direction longitudinale et / ou périphérique.

13. Vêtement de jambe (1) selon la revendication 11 ou 12, **caractérisé en ce que** lorsque le vêtement (1) est porté, au moins une zone (13) d'élasticité accrue ou réduite chevauche dans la direction longitudinale et / ou périphérique les deux chefs musculaires (23, 24) du lambeau musculaire gastrocnémien (22).

14. Vêtement de jambe (1) selon la revendication 2, **caractérisé en ce que** le vêtement (1) comporte hormis la première pièce de tricot de base (2) au moins une bande (14) bicouches, l'au moins un bourrelet (5) qui s'étend le long du tibia s'étendant au moins partiellement dans la bande (14), de préférence entre la première et la deuxième couche de la bande (14).
